# EUROPÄISCHE PATENTANMELDUNG

(11) **EP 0 653 718 A2**
(43) Veröffentlichungstag der Anmeldung: **17.05.1995**
(21) Anmeldenummer: 94117681.0
(22) Anmeldetag: 09.11.1994
(51) Int. Cl.: G06F 19/00, A61B 5/0432

(54) **Vorrichtung und Verfahren zur Erfassung körperspezifischer Messdaten**

(30) Priorität: 16.11.1993 DE 4339188
(71) Anmelder: Müller & Sebastiani Elektronik-GmbH, D-81377 München (DE)
(72) Erfinder: Müller, Peter, D-80798 München (DE); Sebastiani, Oscar, D-80469 München (DE)
(74) Vertreter: Dipl.-Phys.Dr. Manitz Dipl.-Ing. Finsterwald Dipl.-Ing. Grämkow Dipl.-Chem.Dr. Heyn Dipl.-Phys. Rotermund Morgan B.Sc.(Phys.)

(57) **Zusammenfassung**

Die Erfindung betrifft eine tragbare Vorrichtung und ein Verfahren zur Erfassung körperspezifischer Meßdaten, insbesondere EKG- und / oder Blutdruckmeßgeräte, mit einem die Meßdaten liefernden Meßaufnehmer, einer die Meßdaten aufnehmenden, gegen Erschütterungen unempfindlichen Festplatte, einer Einheit zur Ausgabe und / oder Anzeige der erfaßten Meßdaten und einer die Erfassung steuernden Kontrolleinheit.

## Beschreibung

Die Erfindung betrifft eine tragbare Vorrichtung sowie ein Verfahren zur Erfassung körperspezifischer Meßdaten, insbesondere ein EKG- und / oder Blutdruckmeßgerät, mit einem die Meßdaten liefernden Meßaufnehmer, einer die Meßdaten aufnehmenden Speichereinheit, einer Einheit zur Ausgabe und / oder Anzeige der erfaßten Meßdaten und einer die Erfassung steuernden Kontrolleinheit.

Derartige Vorrichtungen werden in der Praxis seit längerer Zeit eingesetzt, um über größere Zeitspannen, insbesondere über vierundzwanzig Stunden, beispielsweise EKG-Daten oder Blutdruckwerte von Patienten zu erfassen.

Dabei ist es problematisch, daß über einen relativ langen Zeitraum eine hohe Anzahl von Daten anfällt, die bis zur nach der Erfassung stattfindenden Ausgabe bzw. Anzeige in der Vorrichtung gespeichert werden müssen. Demzufolge benötigt man ein Speichermedium mit hoher Speicherkapazität, das mit geringem Energieaufwand über lange Zeit betrieben werden können muß. Eine Minimierung des Energieaufwandes ist insbesondere deshalb wünschenswert, da die tragbaren Vorrichtungen der eingangs genannten Art üblicherweise über eine nur begrenzte Energiereserven zur Verfügung stellende mobile Spannungsquelle gespeist werden.

Als Speichereinheit wurden daher bisher vornehmlich Magnetbänder verwendet, die allerdings aufgrund ihrer begrenzten Länge und der hohen anfallenden Datenmenge nur mit einer geringen Bandlaufgeschwindigkeit von beispielsweise 1 mm / sec betrieben werden konnten. Dies führte dazu, daß zum Beispiel bei der Aufnahme von EKG-Signalen mit einer unerwünscht niedrigen Abtastfrequenz von ungefähr 125 Hz gearbeitet werden mußte. Auf diese Weise ließ sich zwar ein Teil der EKG-Signale mit ausreichender Auflösung über einen Zeitraum von vierundzwanzig Stunden aufzeichnen, es war jedoch aufgrund der niedrigen Abtastfrequenz nicht möglich, hochfrequente Signale zu erfassen, wie sie beispielsweise für die Arrhythmieanalyse (gewünschte Abtastfrequenz: 250 Hz) oder die Spätpotentialanalyse (gewünschte Abtastfrequenz: 10 kHz) benötigt werden.

Zudem war das benötigte Bandlaufwerk empfindlich gegen Störungen wie mechanische Unwuchten, Schütteln oder thermische Einflüsse, was jeweils zu einer Aufzeichnung von fehlerhaften Daten führte.

Es ist weiterhin bekannt, anstelle der erwähnten Magnetbänder elektronische Speicherbausteine zu verwenden, in denen die erfaßten Daten speicherbar sind. Hierbei besteht allerdings das Problem, daß die in eine tragbare Vorrichtung der eingangs genannten Art integrierbaren Speicherbausteine eine maximale Speicherkapazität von nur wenigen MB aufweisen, was wiederum nicht genügt, um beispielsweise EKG-Signale mit ausreichender Auflösung für eine später erfolgende umfangreiche Auswertung aufzuzeichnen.

Aus diesem Grund wurde in Vorrichtungen mit elektronischen Speichereinheiten eine Auswerteeinheit integriert, welche die anfallenden Meßdaten bereits in der tragbaren Vorrichtung verarbeitet und lediglich die ermittelten Auswerteparameter im elektronischen Speichermedium ablegt. Da die ermittelten Auswerteparameter einen deutlich geringeren Speicherbereich beanspruchen als die insgesamt während einer Langzeit-Messung anfallenden Meßdaten, kann auf diese Weise ein sinnvoller Betrieb der eingangs genannten Vorrichtung unter Verwendung von elektronischen Speichermedium ermöglicht werden.

Nachteilig an den Vorrichtungen mit elektronischem Speichermedium ist jedoch, daß keine Möglichkeit besteht, den Gesamtverlauf der während einer Langzeitmessung angefallenen Meßdaten zu speichern, so daß es unmöglich ist, diese Meßdaten nach Abschluß der Messung beispielsweise mit einem leistungsfähigen Computer zu verarbeiten. Es war demzufolge nicht möglich, mit diesen Geräten eine von der im Gerät integrierten Auswerteeinheit nicht bewältigbare Spätpotentialanalyse nach der Messung mittels eines leistungsfähigen Computers durchzuführen.

Der Erfindung lag die Aufgabe zugrunde, eine Vorrichtung der eingangs genannten Art so auszubilden, daß die Erfassung von insbesondere hochfrequenten körperspezifischen Meßdaten mit entsprechend hoher Abtastfrequenz über einen längeren Zeitraum möglich ist, wobei die Störanfälligkeit der Vorrichtung minimiert werden soll. Insbesondere soll die Vorrichtung so ausgebildet werden, daß sie mit einem geringen Aufwand an elektrischer Energie betreibbar ist.

Erfindungsgemäß wird diese Aufgabe dadurch gelöst, daß die Speichereinheit als gegen Erschütterungen unempfindliche Festplatte ausgebildet ist, die mit der Vorrichtung über eine genormte Schnittstelle, insbesondere eine PCMCIA-Schnittstelle oder eine ATA-Schnittstelle gekoppelt ist.

Derartige, gegen Erschütterungen unempfindliche Festplatten werden beispielsweise in handelsüblichen Notebook-Computern eingesetzt. Sie sind relativ preiswert und können Erschütterungen von bis zu mehreren 100 g ausgesetzt werden, ohne daß eine Beschädigung auftritt. Zudem eignen sich derartige Festplatten ganz besonders für den Einsatz in tragbaren Vorrichtungen zur Erfassung körperspezifischer Meßdaten, da diese Festplatten mittlerweile im Handel in einem Format von der Größe einer Scheckkarte erhältlich sind. Somit stellen die begrenzten Abmessungen der tragbaren Vorrichtung kein Hindernis beim Einsatz von Festplatten als Speichereinheiten dar.

Durch die erfindungsgemäße Ausgestaltung einer tragbaren Vorrichtung zur Erfassung körperspezifischer Meßdaten ergeben sich eine Reihe von Vorteilen:
- Bei niedrigen Preisen läßt sich eine hohe Speicherkapazität von zum Beispiel 80 MB realisieren. Diese hohe Speicherkapazität ermöglicht das Arbeiten mit einer hohen Abtastrate, wodurch auch hochfrequente Signale mit ausreichender Genauigkeit erfaßt werden können. Insbesondere ist es möglich, in einer erfindungsgemäßen Vorrichtung gespeicherte EKG-Signale für eine nach der Datenerfassung erfolgende Spätpotentialanalyse bzw. eine QRS-Vermessung heranzuziehen.
- Die Meßdaten lassen sich mit einem digitalen Speichermedium wie einer Festplatte mit höherer Genauigkeit aufzeichnen als beispielsweise mit einem aus dem Stand der Technik bekannten Magnetband, welches die Daten analog aufzeichnet.
- Nach erfolgter Meßdatenaufzeichnung ist es erfindungsgemäß nicht nötig, die Vorrichtung weiter mit Energie zu versorgen, da die auf der Festplatte abgelegten Daten auch ohne Energiezufuhr erhalten bleiben. Dies stellt gegenüber der Verwendung von elektronischen Speicherbausteinen einen erheblichen Vorteil dar, da diese auch nach der Erfassung der Meßdaten ständig mit Energie versorgt werden müssen, um zu gewährleisten, daß die in den elektronischen Speichermedien gespeicherten Daten nicht verlorengehen.
- Durch die digitale Speicherung der Daten auf einer Festplatte wird es möglich, diese ohne großen Aufwand innerhalb kurzer Zeit über eine genormte Schnittstelle einer separaten Auswerteeinheit zur Verfügung zu stellen.
- Aufgrund der durch die Festplatte möglichen hohen Speicherkapazität ist es nicht nötig, die erfaßten Meßdaten in komprimierter und damit weniger aussagekräftiger Form zu speichern. Vielmehr ist es möglich, alle erfaßten Meßdaten ohne jegliche Komprimierung auf der Festplatte zu speichern.
- Durch die Kopplung der Festplatte mit der Vorrichtung über eine genormte Schnittstelle ist es möglich, im Handel erhältliche Festplatten ohne Schwierigkeiten zu verwenden, da diese im Regelfall ebenfalls genormte Schnittstellen aufweisen. Außerdem läßt sich auf diese Weise die Festplatte ohne das Erfordernis einer vorherigen Datenübertragung in der Weise mit einer zentralen Auswerteeinheit, insbesondere einem Personalcomputer, koppeln, daß sie von der Auswertungseinheit direkt als Festplattenlaufwerk angesprochen werden kann. Außerdem kann die Festplatte aufgrund der genormten Schnittstelle ohne Probleme gegen ein anderes elektronisches Speichermedium ausgetauscht werden.

Vorzugsweise wird in der erfindungsgemäßen Vorrichtung ein insbesondere als RAM ausgelegter Zwischenspeicher vorgesehen, welcher die erfaßten Meßdaten unmittelbar nach ihrer Erfassung aufnimmt. Dabei wird die Festplatte vorerst nicht angesprochen. Erst wenn im Zwischenspeicher eine vorgegebene Anzahl von Meßdaten vorhanden ist, werden diese vom Zwischenspeicher blockweise auf die Festplatte übertragen. Dieser Vorgang wird immer dann wiederholt, wenn der Zwischenspeicher wieder mit der vorgegebenen Anzahl von neuen Meßdaten beschrieben wurde.

Auf diese Weise ist der wesentliche Vorteil erzielbar, daß die Festplatte immer nur dann in Betrieb genommen werden muß, wenn die Daten blockweise vom Zwischenspeicher auf die Festplatte übertragen werden. Diese Übertragung dauert nur wenige Sekunden, so daß der Einsatz der Festplatte nur einen sehr geringen Energieaufwand bedingt. In der Praxis ist beispielsweise bei der Erfassung von EKG-Signalen bei einem entsprechend ausgelegten Zwischenspeicher nur zweimal pro Stunde eine blockweise Übertragung der Meßdaten vom Zwischenspeicher zur Festplatte nötig. Somit liegt die Betriebszeit der Festplatte während einer vierundzwanzig Stunden dauernden Meßdatenerfassung lediglich im Minutenbereich. Auf diese Weise wird eine äußerst energiesparenden Betriebsweise der gesamten Vorrichtung ermöglicht.

Bei einer vorteilhaften Ausführungsform der Erfindung weist die Festplatte eine Speicherkapazität von 80 MB und der Zwischenspeicher - falls vorhanden - eine Speicherkapazität von ungefähr 2 MB auf. Je nach Anforderung lassen sich jedoch auch andere Speicherkapazitäten realisieren.

Besonders vorteilhaft ist es, wenn die Festplatte über eine mechanisch-elektronische Steckverbindung derart in der Vorrichtung angeordnet ist, daß sie ohne Aufwand von der Vorrichtung abkoppelbar ist. Gerade bei der Verwendung der erwähnten PCMCIA- oder ATA-Schnittstellen kann dann erreicht werden, daß keine separaten mechanischen Halterungselemente für die Festplatte in der Vorrichtung vorgesehen werden müssen. Weiterhin kann die Festplatte so in der Vorrichtung angeordnet werden, daß sie von außen ohne Schwierigkeiten zugänglich ist und aus der Vorrichtung entnommen werden kann.

Eine derartige herausnehmbare Festplatte kann beispielsweise nach der Meßdatenerfassung aus der Vorrichtung entnommen werden und entweder einer zentralen Auswertestelle zugeleitet oder direkt mittels eines Auswerte-Computers ausgewertet werden. Über eine PCMCIA- oder ATA-Schnittstelle kann die Festplatte in der Weise an den Auswerte-Computer angeschlossen werden, daß dieser die angeschlossene Festplatte direkt als Laufwerk ansprechen kann. Eine elektronische Übertragung der Daten von der herausnehmbaren Festplatte auf eine im Auswerte-Computer vorhandene Festplatte ist dann nicht mehr nötig.

Anstelle der Festplatte kann auch ein elektronisches Speichermedium, insbesondere ein RAM oder ein Flash-EPROM Verwendung finden. Beim Einsatz eines Flash-EPROM ergibt sich gegenüber herkömmlichen elektronischen Speichermedien der Vorteil, daß das Speichermedium nach der Datenerfassung nicht mehr mit elektrischer Energie versorgt werden muß.

Bei der Verwendung von PCMCIA- oder ATA-Schnittstellen ist es weiterhin auch möglich, in der Vorrichtung wahlweise anstelle der Festplatte eine einen RAM-Speicher aufweisende PCMCIA- oder ATA-Memory-Karte oder ein anderes, einen PCMCIA- oder ATA-Anschluß aufweisendes Speichermedium zu verwenden. Dadurch läßt sich die Vorrichtung in verschiedenen Anwendungsfällen außerordentlich flexibel einsetzen.

Vorzugsweise ist die erfindungsgemäße Vorrichtung zusätzlich mit einer seriellen und / oder parallelen Schnittstelle versehen, über die die auf der Festplatte gespeicherten Daten ausgegeben, insbesondere an einen Auswerte-Computer übermittelt werden können.

Weiterhin ist es möglich, in der erfindungsgemäßen Vorrichtung eine Auswerteeinheit vorzusehen, welche aus den erfaßten Meßdaten bestimmte Auswerteparameter berechnet und diese gegebenenfalls zusätzlich zu den erfaßten Meßdaten auf der Festplatte speichert. So können bestimmte, für die Auswertung erforderliche Berechnungen von dem meist stationären Auswerte-Computer in die tragbare Vorrichtung selbst verlagert werden.

Dadurch, daß es erfindungsgemäß möglich ist, über lange Zeiträume mit einer hohen Abtastfrequenz zu arbeiten, können EKG-Signale mit so großer Genauigkeit aufgenommen werden, daß eine nachfolgende Arrhythmie- oder Spätpotentialanalyse erfolgen kann. Für eine Arrhythmieanalyse wird vorzugsweise mit einer Abtastfrequenz von 250 Hz und für eine Spätpotentialanalyse mit einer Abtastfrequenz von 10 kHz gearbeitet.

Durch die Erfindung wird ferner der Vorteil erreicht, daß die auf der Festplatte gespeicherten Daten von einer Zentraleinheit ausgewertet werden können, während die tragbare Vorrichtung bereits wieder anderweitig verwendet wird, da die Festplatte nicht fest mit der tragbaren Vorrichtung verbunden ist.

Weitere bevorzugte Ausführungsformen der Erfindung sind in den Unteransprüchen angegeben.

Die Erfindung wird nachfolgend anhand der einzigen Figur beschrieben; es zeigt dabei:

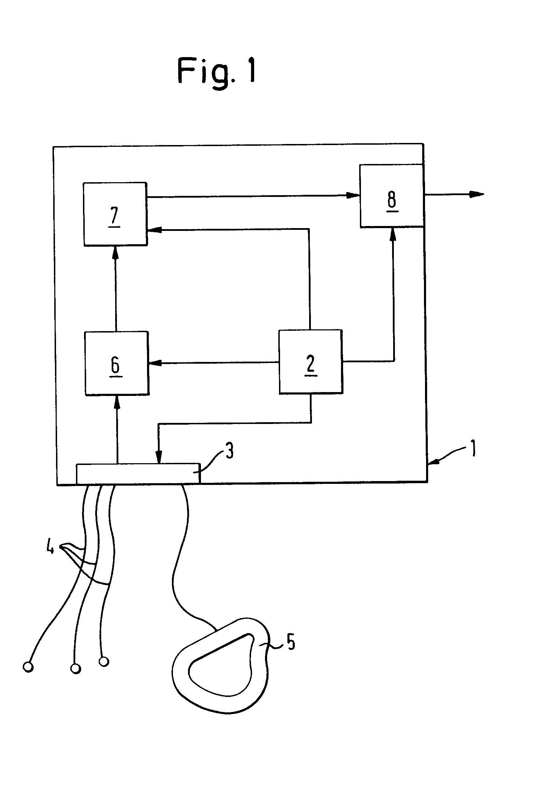

Figur 1 den schematischen Aufbau einer erfindungsgemäßen Vorrichtung.

Die Gesamtvorrichtung 1 weist eine zentrale Kontrolleinheit 2 auf, welche eine Schnittstelleneinheit 3 ansteuert.

Über die Schnittstelleneinheit 3 werden EKG-Meßaufnehmer 4 sowie ein Blutdruck-Meßaufnehmer 5 an der Vorrichtung 1 angeschlossen. Die von den Meßaufnehmern 4, 5 gelieferten Meßdaten werden zuerst kontinuierlich in einen von der Kontrolleinheit 2 angesteuerten Zwischenspeicher 6 übertragen.

Sobald der Zwischenspeicher 6 mit Meßdaten gefüllt ist, beginnt eine blockweise Übertragung dieser Meßdaten aus dem Zwischenspeicher in einen ebenfalls von der Kontrolleinheit 2 angesteuerten Festplattenspeicher 7. Während der Übertragung der Meßdaten vom Zwischenspeicher 6 in den Festplattenspeicher 7 wird die kontinuierliche Erfassung von Meßdaten und deren Übertragung in den Zwischenspeicher 6 nicht unterbrochen.

Nach Abschluß der Meßdatenerfassung können die Meßdaten vom Festplattenspeicher 7 über eine ebenfalls von der Kontrolleinheit 2 angesteuerte Ausgabeeinheit 8 ausgelesen und an einen nicht dargestellten Auswerte-Computer übertragen werden.

## Patentansprüche

1. Tragbare Vorrichtung zur Erfassung körperspezifischer Meßdaten, insbesondere EKG-und/oder Blutdruckmeßgerät, mit einem die Meßdaten liefernden Meßaufnehmer (4, 5), einer die Meßdaten aufnehmenden Speichereinheit (7), einer Einheit (8) zur Ausgabe und / oder Anzeige der erfaßten Meßdaten und einer die Erfassung steuernden Kontrolleinheit (2), dadurch gekennzeichnet,
daß die Speichereinheit (7) als gegen Erschütterungen unempfindliche Festplatte ausgebildet ist, die mit der Vorrichtung (1) über eine genormte Schnittstelle, insbesondere eine PCMCIA-Schnittstelle oder eine ATA-Schnittstelle gekoppelt ist.

2. Vorrichtung nach Anspruch 1,
dadurch gekennzeichnet,
daß die Festplatte (7) über eine mechanisch-elektronische Steckverbindung derart in der Vorrichtung (1) angeordnet ist, daß sie ohne Aufwand von der Vorrichtung (1) abkoppelbar ist.

3. Vorrichtung nach Anspruch 1 oder 2,
dadurch gekennzeichnet,
daß ein eine geringere Speicherkapazität als die Festplatte (7) aufweisender Zwischenspeicher (6) zur kontinuierlichen Aufnahme und anschließenden blockweisen Weiterleitung der Meßdaten an die Festplatte (7) vorgesehen ist.

4. Vorrichtung nach Anspruch 3,
dadurch gekennzeichnet,
daß der Zwischenspeicher (6) als RAM ausgelegt ist und vorzugsweise eine Speicherkapazität von 2 MB aufweist und daß die Festplatte (7) eine Speicherkapazität von 80 MB aufweist.

5. Vorrichtung nach Anspruch 1 und 2,
dadurch gekennzeichnet,
daß eine serielle und / oder parallele Schnittstelle (8) zur Ausgabe von Daten und eine Auswerteeinheit zur Berechnung von vorgebbaren, auf der Festplatte (7) speicherbaren Parametern vorgesehen ist.

6. Vorrichtung nach Anspruch 1,
dadurch gekennzeichnet,
daß der Meßaufnehmer (4) zur Aufnahme von Spätpotentialen und / oder zur QRS-Vermessung geeignet ist.

7. Vorrichtung nach Anspruch 1,
dadurch gekennzeichnet,
daß anstelle der Festplatte (7) ein elektronisches Speichermedium, insbesondere ein RAM-Speicher oder ein Flash-EPROM, eine PCMCIA-Memory-Karte oder eine ATA-Memory-Karte verwendet ist.

8. Verfahren zur mobilen Erfassung von körperspezifischen Meßdaten mit einer Vorrichtung nach einem der Ansprüche 1 bis 7,
dadurch gekennzeichnet,
daß die Meßdaten während der Erfassung kontinuierlich oder blockweise auf der Festplatte abgespeichert werden, so daß die erfaßten Signale nach Beendigung des Meßvorgangs aus den abgespeicherten Daten zum Zweck einer nachträglichen Auswertung rekonstruierbar sind, wobei insbesondere alle erfaßten Meßdaten ohne vorherige Komprimierung auf der Festplatte (7) gespeichert werden.

9. Verfahren nach Anspruch 8,
dadurch gekennzeichnet,
daß die Festplatte (7) nach der Meßdatenerfassung aus der Vorrichtung (1) herausgenommen und an einen Computer angeschlossen wird, welcher die erfaßten Meßdaten auswertet, und daß die herausnehmbare Festplatte (7) insbesondere in der Weise an den Computer ankoppelbar ist, daß sie von diesem direkt als Festplattenlaufwerk angesprochen werden kann.

10. Verfahren nach Anspruch 8,
dadurch gekennzeichnet,
daß während der kontinuierlich über einen Zeitraum von vierundzwanzig Stunden oder über einen längeren, vorzugsweise mehrere Tage dauerenden Zeitraum erfolgenden Meßdatenerfassung ungefähr zweimal pro Stunde eine blockweise Übertragung der Meßdaten vom Zwischenspeicher (6) zur Festplatte (7) stattfindet.

11. Verfahren nach Anspruch 8,
dadurch gekennzeichnet,
daß die Meßdaten zur Arrhythmieanalyse mit einer Abtastfrequenz von 250 Hz und die Meßdaten zur Spätpotentialanalyse mit einer Abtastfrequenz von 10 kHz erfaßt werden.
